(19) Europäisches Patentamt
European Patent Office
Office européen des brevets

(11) Publication number: 0 055 716 B1

(12) # EUROPEAN PATENT SPECIFICATION

(45) Date of publication of patent specification: 07.11.84

(51) Int. Cl.$^3$: A 61 K 31/71, A 61 K 31/425, A 61 K 31/52, A 61 K 31/35

(21) Application number: 81901684.1

(22) Date of filing: 23.06.81

(86) International application number: PCT/AU81/00077

(87) International publication number: WO 82/00003 07.01.82 Gazette 82/01

(54) **AMPLIFIED ANTIBIOTIC AND ANTITUMOR AGENTS.**

(30) Priority: 24.06.80 AU 4170/80

(43) Date of publication of application: 14.07.82 Bulletin 82/28

(45) Publication of the grant of the patent: 07.11.84 Bulletin 84/45

(84) Designated Contracting States: CH DE FR GB LI NL SE

(56) References cited:
AU-A- 35 175
AU-B- 11 159
AU-B- 42 296
AU-B- 52 825
AU-B- 84 888
CA-A-1 091 583
FR-A-2 461 716
US-A-4 238 391

Excerpta Medica, No. 80224990, Iseki et al, "Effects of caffeine on neocarzinostatin-induced inhibition of cell cycle traverse in hela-S3 cells", Cancer Research, vol. 40, no. 10, issued 1980, Baltimore, Maryland. U.S.A.

(73) Proprietor: COMMONWEALTH SCIENTIFIC AND INDUSTRIAL RESEARCH ORGANIZATION
Limestone Avenue
Campbell Australian Capital Territory 2601 (AU)

(72) Inventor: GRIGG, Geoffrey Walter
352 Burns Bay Road
Lane Cove, N.S.W. 2066 (AU)
Inventor: LAMBERTON, John
10 Tuxen Court
East Brighton, Vic. 3187 (AU)

(74) Representative: Gordon, Richard John Albert et al
F.J. CLEVELAND & COMPANY 40-43, Chancery Lane
London WC2A 1JQ (GB)

(56) References cited:
Chemical Abstracts, vol. 73, no. 21, issued 1970, Grigg, G.W., "Amplification of Phleomycin-induced death and DNA breakdown by caffeine in E.Coli", Abstract no. 73106635H, Journal of Molecular Genetics" Series 107, issue 2, pages 162-72

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

(56) References cited:
Chemical Abstracts, vol. 75, no. 17, issued 1971, October 25, Grigg, G.W., "Effect of coumarin, thiopurines and pyronin Y on amplification of phleomycin-induced death and dexyribonucleic acid breakdown in E.Coli", abstract no. 75106530M, Journal of Bacteriology, Series 107, issue 3, page 599-609
CHEMICAL ABSTRACTS, VOL: 82, No. 11, March 17, 1975, page 78, column 2, abstract no. 68750t, Columbus, Ohio, US, A.M. ANGYAL et al.: "Purines as amplifiers of the antibiotic activity of phleomycin against Escherichia coli B."

**Description**

### Technical Field

The present invention relates to a method for amplifying the activity of certain compounds having antibiotic and antitumor properties.

### Background Art

It has been known for some time that certain compounds obtained by the culture of *streptomyces verticillus* and related species show antibiotic and antitumor properties. These include phleomycin, bleomycin and tallysomycin. Subsequent research showed that each of these materials were in fact mixtures of amides of the relevant acid i.e. phleomycinic acid, bleomycinic acid and tallysomycinic acid. The components of the originally isolated mixtures have been isolated and new amides have since been synthesised.

While many of the known amides of phleomycinic, bleomycinic and tallysomycinic acids are known to have antibacterial and antitumor properties it is also known that many of them have unfortunate side effects such as nephrotoxicity.

The present applicants Australian patent number 496,392 and Australian patent application 35175/78 disclose that certain compounds are capable of amplifying the activity of certain of the above amides. This has allowed use of these compounds in reduced, and therefore less harmful doses. The present invention concerns an extension of this concept.

In particular Australian Patent Application No. 35175/78 discloses the use of Pyronine Y as an amplifier for phleomycin and bleomycin for both antibiotic and anti-tumour pharmaceutical preparations. The amplification of phleomycin induced death and DNA breakdown by caffeine in *E coli* is also described in Mol. Gen. Genet. 1970 107(2) Pages 162—172.

According to one aspect of the invention there is provided a pharmaceutical composition comprising a tallysomycinic acid amide wherein the amine moiety is selected from compounds having the structural formulae:-

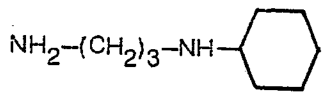

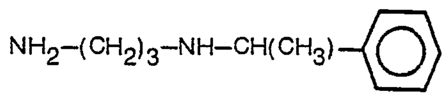

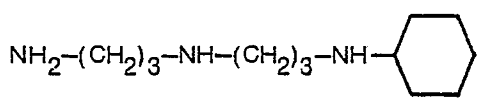

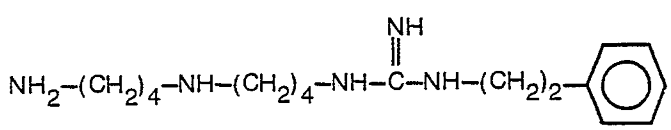

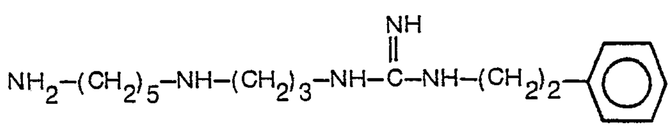

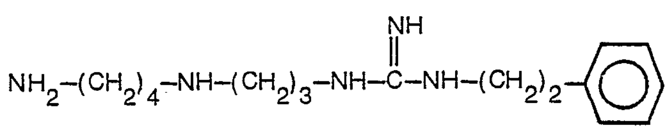

and an amplifying compound selected from caffeine, dimethylthiocyanine and Pyronine Y.

In another aspect of the invention there is provided a pharmaceutical composition comprising an amide of phleomycinic acid, bleomycinic acid or tallysomycinic acid wherein the amine moiety is selected from the compounds having the structural formulae:—

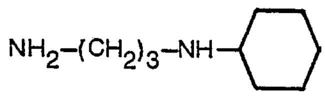

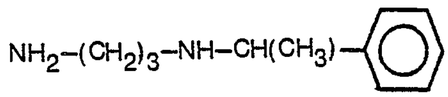

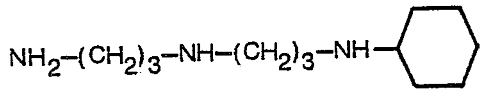

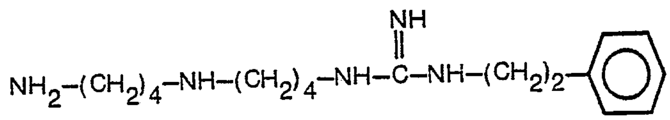

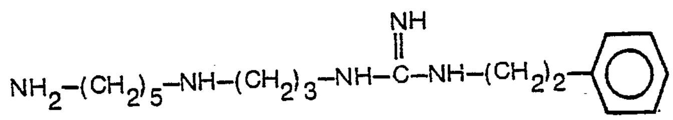

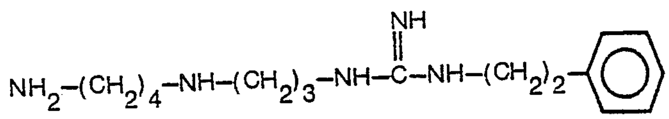

and the amplifying compound dimethylthiocyanine.

In a further aspect of the invention there is provided a pharmaceutical composition for use as an anti-tumour agent comprising an amide phleomycinic acid, bleomycinic acid or tallysomycinic acid wherein the amine moiety is selected from compounds having the structural formulae:—

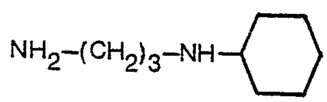

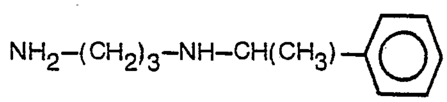

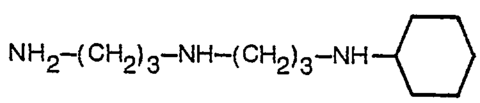

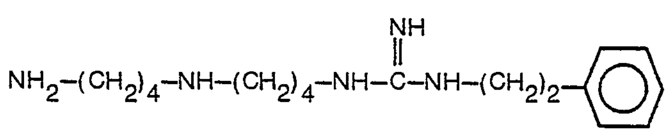

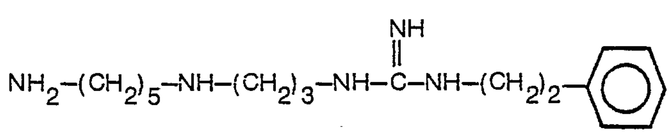

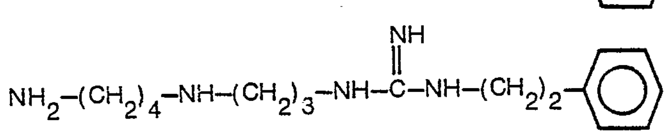

and an amplifying compound selected from caffeine and dimethylthiocyanine.

Dimethylthiocyanine is

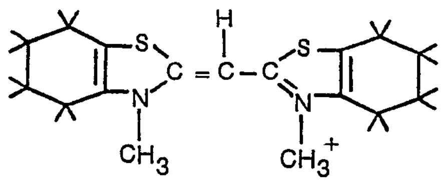

The acid residues of the amides to which the present invention relates are known and their structure has been published and for this reason their structural formula is not reproduced here. In each of these compounds there is a terminal carboxylic group which in the compounds to which the present invention relates has been reacted with an amine moiety to give an amide compound. The amides may be obtained semi synthetically by culturing *Streptomyces verticillus* in the presence of an appropriate amine compound which will be incorporated into the amide produced by the organism or alternatively, some other amide may be synthetically manipulated to produce the desired compound.

It should be noted that the phleomycin and bleomycin referred to in the prior art have been a mixture of amides but these known mixtures principally contained amines other than those to which the present invention relates. It is not possible to predict *a priori* the effect of any given amplifier upon any given amide. The selection according to the present invention is based upon the degree of amplification achieved by the amplifier on the designated amide and the nephrotoxicity for the combination of amplifier and amide.

The amplifying compounds are caffeine, dimethylthiocyanine and Pyronine Y. Caffeine is 1, 3, 7 trimethyl xanthene and has the elemental formula $C_8H_{10}O_2N_4 \cdot H_2O$ and Pyronine Y is a brand name of 6-(dimethylamino)-3H-xanthene-3-ylidene-dimethylammonium chloride.

The weight ratio of amide to activator to achieve optimal activation must be determined by routine testing on a case by case basis. It has been found experimentally that ratios of from 1:0.002 up to 1:30 and above have given significant enhancement under the particular circumstances of the experiment.

The amides of use in this invention have been given abbreviated names for ease of reference and in this specification the following abbreviations will be used:—

| Amine Moiety | Abbreviation |
|---|---|
| 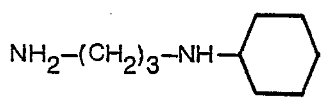 | CHP |
| 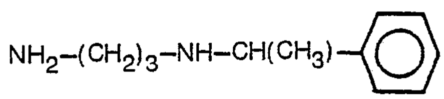 | PEP |
| 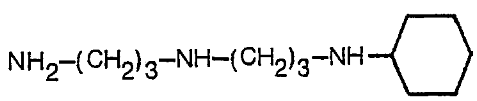 | CHPP |
| 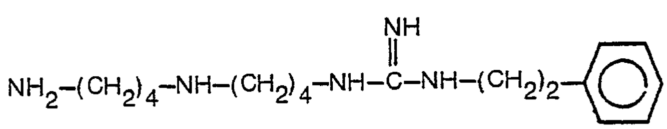 | AAGPe |

Thus bleomycin having the CHP amine moiety is called bleomycin CHP and the other acid amides are similarly described.

The amide compound may be administered simultaneously with the activator compound or alternatively it may be administered some time in advance of the activator compound.

## Best Mode of Carrying out the Invention

The antibacterial and antitumour activity of the amide/activator combinations of this invention will now be illustrated in detail by reference to specific examples.

### Method

Random bred albino Sprague Dawley rats were used with Walker 256 carcinoma Sarcoma. Tumour passages were effected by intraperitoneal injection of $10^6$ cells which had been prepared by aspirating several ml of ascites fluid from a rat which had been carrying an ascites tumour for 7 days.

This was given two 2-minute treatments with Boyle's fluid (9 vols. 0.83% $NH_4Cl$, 1 vol. Tris buffer, pH7.2). The cell suspension was counted on a haemocytometer using Turk's fluid and then diluted with Eagle's medium to $10^7$ live cells per ml, and then 0.1 ml was injected intraperitoneally. Bacto-fluid Thiolglycollate Medium was used to test for contamination.

Solutions of the phleomycins, bleomycins or tallysomycins were made up in phosphate-buffered saline at pH7.2 in daily doses at the commencement of each 5 day injection period and were kept frozen until the commencement of each day's injections. The injections were given at 9 a.m. and 4 p.m. Bulk solutions of amplifier were stored at room temperature and were injected 30 minutes after the active compound. In most experiments several levels of active compound and amplifier were given both alone and in combination, and several groups of test animals were injected with buffered saline alone, i.e. with the vehicle for the active compound and amplifier.

Test groups usually comprised 7 or 8 animals. Procedures were based closely on those detailed by Giran et al (1972). Cancer Chemotherapy Reports, September 1972, Vol. 3 No. 2 Part 3. All groups were retained until their median survival time had been marked and a T/C (2) equal to or greater than 125 was considered a positive result.

$$T/C = \frac{\text{median survival time (M.S.T.) of test group}}{\text{median survival time (M.S.T.) of control group}}$$

T/C (1) is the T/C where the control group is the saline vehicle only group and

T/C (2) is the T/C where the control group is whichever of the relative unamplified amide group or the amplifier only group has the higher M.S.T. value.

The results are given in Table 1. The table also includes results for trials in which the potentiator was administered (i) via the drinking water, and (ii) from Alzet minipumps implanted under the skin.

Tables 2 and 3 give results for similar tests carried out using mice infected with Ehrlich's Ascites Tumor, or with Mouse Sarcoma 180.

TABLE 1

| TREATMENT | LEVEL OF PHLEOMYCIN (uKD) | LEVEL OF AMPLIFIER (uKD) | M.S.T. (DAYS) | (1) | T/C (2) |
|---|---|---|---|---|---|
| EXPERIMENT 1A | | | | | |
| Phleomycin PEP | 3840 | — | 24.0 | 266 | — |
| Phleomycin PEP + Caffeine | 3840 | 106.1 | 100 | 1111 | (416)* |
| Phleomycin PEP + Caffeine | 3840 | 10.61 | 100 | 1111 | (416)* |
| Phleomycin PEP | 1280 | — | 21.0 | 233 | — |
| Phleomycin PEP + Caffeine | 1280 | 106.1 | 100 | 1111 | (476)* |
| Phleomycin PEP + Caffeine | 1280 | 10.61 | 60.0 | 667 | (285)* |
| Caffeine | — | 106.1 | 8.0 | 89 | — |
| Caffeine | — | 10.61 | 8.0 | 89 | — |
| Saline Vehicle Only | — | — | 9.0 | — | — |
| EXPERIMENT 1B | | | | | |
| Phleomycin CHP | 1000 | — | 21.0 | 280 | — |
| Phleomycin CHP + Caffeine | 1000 | 1061 | 21.0 | 280 | (100) |
| Phleomycin CHP + Caffeine | 1000 | 10.61 | 16.0 | 213 | (76) |
| Phleomycin CHP | 250 | — | 9.0 | 120 | — |
| Phleomycin CHP + Caffeine | 250 | 1061 | 9.0 | 120 | (100) |
| Phleomycin CHP + Caffeine | 250 | 10.61 | 100.0 | 1330 | (1111)* |
| Phleomycin CHP | 125 | — | 9.0 | 120 | — |
| Phleomycin CHP + Caffeine | 125 | 1061 | 7.0 | 93 | (78) |
| Phleomycin CHP + Caffeine | 125 | 10.61 | 100.0 | 1330 | (1111)* |
| Caffeine | — | 1061 | 8.0 | 107 | — |
| Caffeine | — | 10.61 | 7.0 | 93 | — |
| Saline Vehicle Only | — | — | 7.5 | — | — |
| EXPERIMENT 1C | | | | | |
| Phleomycin CHP | 1000 | — | 19.0 | 237 | — |
| Phleomycin CHP | 500 | — | 10.0 | 125 | — |
| Phleomycin CHP + Dimethylthiocyanine | 500 | 675 | 18.0 | 225 | (180)* |
| Phleomycin CHP | 250 | — | 9.0 | 112 | — |
| Phleomycin CHP + Dimethylthiocyanine | 250 | 675 | 17.0 | 212 | (188)* |

TABLE 1 (continued)

| TREATMENT | LEVEL OF PHLEOMYCIN (uKD) | LEVEL OF AMPLIFIER (uKD) | M.S.T. (DAYS) | (1) | T/C (2) |
|---|---|---|---|---|---|
| EXPERIMENT 1C | | | | | |
| Phleomycin CHP | 125 | — | 9.0 | 112 | — |
| Phleomycin CHP + Dimethylthiocyanine | 125 | 675 | 14.0 | 175 | (155)* |
| Dimethylthiocyanine | — | 675 | 9.0 | 112 | — |
| Saline Vehicle Only | — | — | 8.0 | — | — |
| EXPERIMENT 1D | | | | | |
| Phleomycin CHP | 250 | — | 14.0 | 133 | — |
| Phleomycin CHP + Caffeine in Minipumps | 250 | 106.1 | 100.0 | 952 | (714)* |
| Phleomycin CHP + Caffeine in Minipumps | 250 | 10.61 | 20.5 | 195 | (146)* |
| Phleomycin CHP | 125 | — | 13.5 | 129 | — |
| Phleomycin CHP + Caffeine in Minipumps | 125 | 106.1 | 12.0 | 114 | (88) |
| Phleomycin CHP + Caffeine in Minipumps | 125 | 10.61 | 26.0 | 248 | (193)* |
| Caffeine in Minipumps | — | 106.1 | 10.0 | 95 | — |
| Caffeine in Minipumps | — | 10.61 | 10.0 | 95 | — |
| Saline Vehicle Only | — | — | 10.5 | — | — |
| EXPERIMENT 1E | | | | | |
| Phleomycin AAGPE | 300 | — | 10.5 | 175 | — |
| Phleomycin AAGPE + Dimethylthiocyanine | 300 | 675 | 14.0 | 233 | (133)* |
| Phleomycin AAGPE | 150 | — | 10.5 | 175 | — |
| Phleomycin AAGPE + Dimethylthiocyanine | 150 | 675 | 17.5 | 292 | (167)* |
| Phleomycin AAGPE | 75 | — | 8.5 | 141 | — |
| Phleomycin AAGPE + Dimethylthiocyanine | 75 | 675 | 11.5 | 191 | (135)* |
| Phleomycin AAGPE | 38 | — | 7.5 | 125 | — |
| Phleomycin AAGPE + Dimethylthiocyanine | 38 | 675 | 9.5 | 158 | (127)* |
| Dimethylthiocyanine | — | 675 | 6.0 | 100 | — |
| Saline Vehicle Only | — | — | 6.0 | — | — |

TABLE 1 (continued)

| TREATMENT | LEVEL OF PHLEOMYCIN (uKD) | LEVEL OF AMPLIFIER (uKD) | M.S.T. (DAYS) | (1) | T/C (2) |
|---|---|---|---|---|---|
| EXPERIMENT 1F | | | | | |
| Bleomycin CHP alone | 1000 | — | 30.0 | 300 | |
| Bleomycin CHP + Dimethylthiocyanine | 1000 | 675 | 100 | 1000 | (333)* |
| Saline Vehicle Only | — | — | 10.0 | — | — |

TABLE 2

MOUSE — EHRLICH'S ASCITES TUMOR
Antibiotic and Amplifier by Intraperitoneal Injection
(Unless Indicated Otherwise)

| TREATMENT | LEVEL OF PHLEOMYCIN (uKD) | LEVEL OF AMPLIFIER (uKD) | M.S.T. (DAYS) | (1) | T/C (2) |
|---|---|---|---|---|---|
| EXPERIMENT 2A | | | | | |
| Phleomycin AAGPE | 300 | — | 29.5 | 131 | — |
| Phleomycin AAGPE + Caffeine | 300 | 106.1 | 44.5 | 197 | (150)* |
| Phleomycin AAGPE + Caffeine | 300 | 10.61 | 30.5 | 135 | (103) |
| Phleomycin AAGPE | 150 | — | 26.0 | 115 | — |
| Phleomycin AAGPE + Caffeine | 150 | 106.1 | 27.0 | 120 | (104) |
| Phleomycin AAGPE + Caffeine | 150 | 10.61 | 52.0 | 231 | (200)* |
| Caffeine | — | 106.1 | 21.0 | 93 | — |
| Caffeine | — | 10.61 | 21.0 | 93 | — |
| Saline Vehicle Only | — | — | 22.5 | — | — |
| Phleomycin AAGPE | 600 | — | 21.0 | 113 | — |
| EXPERIMENT 2B | | | | | |
| Phleomycin AAGPE | 300 | — | 20.5 | 110 | — |
| Phleomycin AAGPE + Caffeine in drinking water | 300 | 1061 | 20.0 | 108 | (97) |
| Phleomycin AAGPE + Caffeine in drinking water | 300 | 106.1 | 20.5 | 110 | — |
| Phleomycin AAGPE + Caffeine in drinking water | 300 | 10.61 | 20.0 | 108 | (97) |
| Phleomycin AAGPE | 150 | — | 19.0 | 102 | — |
| Phleomycin AAGPE + Caffeine in drinking water | 150 | 1061 | 24.5 | 132 | (129)* |
| Phleomycin AAGPE + Caffeine in drinking water | 150 | 106.1 | 20.5 | 110 | (108) |
| Phleomycin AAGPE + Caffeine in drinking water | 150 | 10.61 | 19.0 | 102 | — |
| Caffeine in drinking water | | 1061 | 20.0 | 108 | — |
| Caffeine in drinking water | — | 106.1 | 21.0 | 113 | — |
| Caffeine in drinking water | — | 10.61 | 22.0 | 119 | — |
| Saline Vehicle Only | — | — | 18.5 | — | — |

TABLE 2 (cont.)

MOUSE—EHRLICH'S ASCITES TUMOR
Antibiotic and Amplifier by Intraperitoneal Injection
(Unless Otherwise Indicated)

| TREATMENT | LEVEL OF PHLEOMYCIN (uKD) | LEVEL OF AMPLIFIER (uKD) | M.S.T. (DAYS) | (1) | T/C (2) |
|---|---|---|---|---|---|
| EXPERIMENT 2C | | | | | |
| Phleomycin $AC_5AC_3GPe$ | 2700 | — | 32.0 | 171 | — |
| Phleomycin $AC_5AC_3GPe$ + Dimethylthiocyanine | 2700 | 2025 | 24.0 | 133 | (75) |
| Phleomycin $AC_5AC_3GPe$ + Dimethylthiocyanine | 2700 | 675 | 40.0 | 222 | (125)* |
| Phleomycin $AC_5AC_3GPe$ | 900 | — | 27.0 | 150 | — |
| Phleomycin $AC_5AC_3GPe$ + Dimethylthiocyanine | 900 | 2025 | 33.0 | 183 | (122) |
| Phleomycin $AC_5AC_3GPe$ + Dimethylthiocyanine | 900 | 675 | 58.0 | 322 | (214)* |
| Phleomycin $AC_5AC_3GPe$ | 300 | — | 24.0 | 133 | — |
| Phleomycin $AC_5AC_3GPe$ + Dimethylthiocyanine | 300 | 2025 | 30.0 | 166 | (125)* |
| Phleomycin $AC_5AC_3GPe$ + Dimethylthiocyanine | 300 | 675 | 22.0 | 122 | (91) |
| Phleomycin $AC_5AC_3GPe$ | 100 | — | 20.0 | 111 | — |
| Phleomycin $AC_5AC_3GPe$ + Dimethylthiocyanine | 100 | 2025 | 20.0 | 111 | (100) |
| Phleomycin $AC_5AC_3GPe$ + Dimethylthiocyanine | 100 | 675 | 18.0 | 100 | (90) |
| Phleomycin $AC_5AC_3GPe$ | 33 | — | 20.0 | 111 | — |
| Phleomycin $AC_5AC_3GPe$ + Dimethylthiocyanine | 33 | 2025 | 20.0 | 111 | (100) |
| Phleomycin $AC_5AC_3GPe$ + Dimethylthiocyanine | 33 | 675 | 20.0 | 111 | (100) |
| Dimethylthiocyanine | — | 2025 | 20.0 | 111 | — |
| | — | 675 | 20.0 | 111 | — |
| Saline Vehicle Only | — | — | 18.0 | — | — |

TABLE 3

MOUSE SARCOMA 180
Antibiotic and Amplifier by Intraperitoneal Injection
(Unless Indicated Otherwise)

| TREATMENT | LEVEL OF BLEOMYCIN (uKD) | LEVEL OF AMPLIFIER (uKD) | M.S.T. (DAYS) | (1) | T/C (2) |
|---|---|---|---|---|---|
| EXPERIMENT 3A | | | | | |
| Bleomycin CHP | 1000 | — | 25.0 | 156 | — |
| Bleomycin CHP + Caffeine | 1000 | 1942 | 61.0 | 381 | (244)* |
| Bleomycin CHP | 250 | — | 18.0 | 112 | — |
| Bleomycin CHP + Caffeine | 250 | 1942 | 29.0 | 181 | (161)* |
| Caffeine | — | 1942 | 17.0 | 106 | — |
| Saline Vehicle Only | — | — | 16.0 | — | — |
| EXPERIMENT 3B | | | | | |
| TREATMENT | LEVEL OF PHLEOMYCIN (uKD) | LEVEL OF AMPLIFIER (uKD) | M.S.T. (DAYS) | (1) | T/C (2) |
| Phleomycin AAGPE | 300 | — | 18.0 | 106 | — |
| Phleomycin AAGPE + Dimethylthiocyanine | 300 | 675 | 24.0 | 141 | (133)* |
| Dimethylthiocyanine | — | 675 | 17.0 | 100 | — |
| Saline Vehicle Only | — | — | 17.0 | — | — |
| EXPERIMENT 3C | | | | | |
| Phleomycin CHP | 300 | — | 17.0 | 100 | — |
| Phleomycin CHP + Dimethylthiocyanine | 300 | 675 | 22.0 | 129 | (129)* |
| Phleomycin CHP | 100 | — | 15.0 | 88 | — |
| Phleomycin CHP + Dimethylthiocyanine | 100 | 675 | 20.0 | 118 | (133)* |
| Dimethylthiocyanine | — | 675 | 17.0 | 100 | — |
| Saline Vehicle Only | — | — | 17.0 | — | — |

## Claims

1. A pharmaceutical composition comprising a tallysomycinic acid amide wherein the amine moiety is selected from compounds having the structural formulae:—

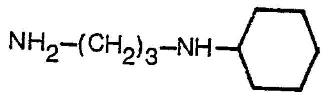

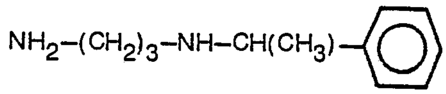

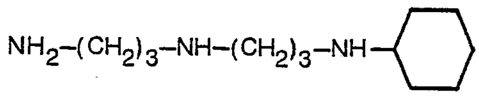

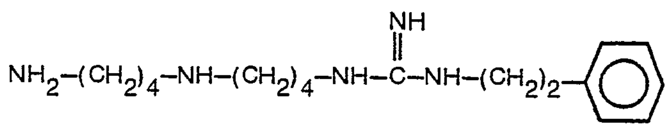

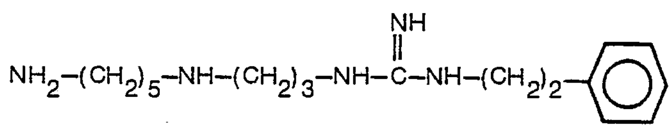

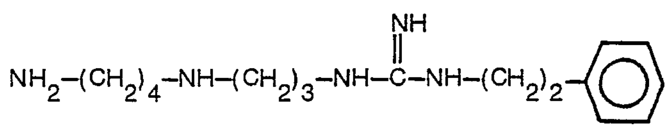

and an amplifying compound selected from caffeine, dimethylthiocyanine and Pyronine Y.

2. A pharmaceutical composition comprising an amide of phleomycinic acid, bleomycinic acid or tallysomycinic acid wherein the amine moiety is selected from the compounds having the structural formulae:—

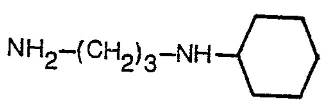

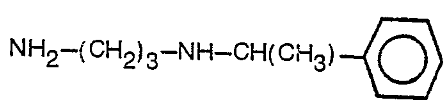

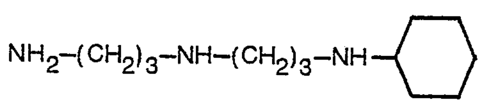

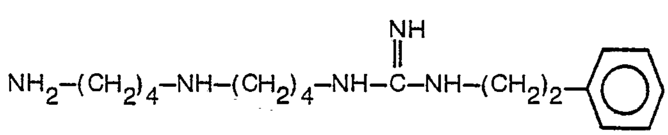

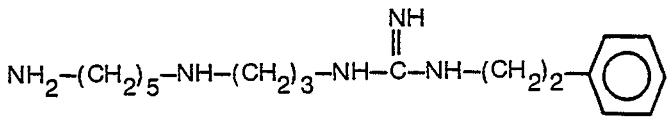

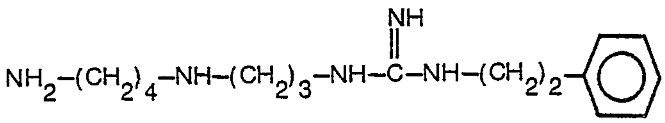

and the amplifying compound dimethylthiocyanine.

3. A pharmaceutical composition for use as an anti-tumour agent comprising an amide phleomycinic acid, bleomycinic acid or tallysomycinic acid wherein the amine moiety is selected from compounds having the structural formulae:—

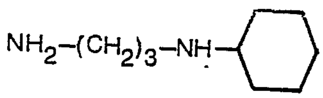

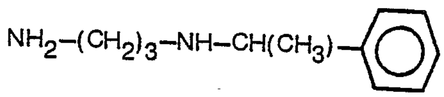

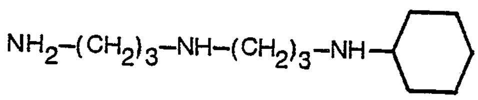

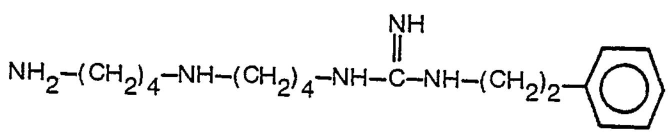

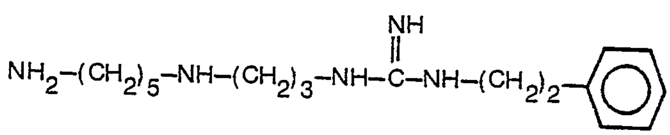

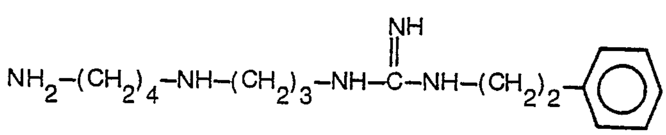

and an amplifying compound selected from caffeine and dimethylthiocyanine.

4. A pharmaceutical composition as claimed in either of claims 2 or 3 in which the amide is an amide of pleomycinic acid.

5. A pharmaceutical composition as claimed in either of claims 2 or 3 in which the amide is an amide of bleomycinic acid.

6. A pharmaceutical composition as claimed in any one preceding claim in which the amide and the amplifying compound are adapted for administration in a weight ratio of from 1:0.002 to 1:30.

7. A pharmaceutical composition as claimed in any preceding claim in which the amine moiety is selected from compounds having the structure formulae:—

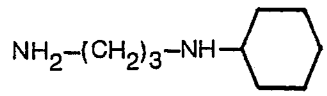

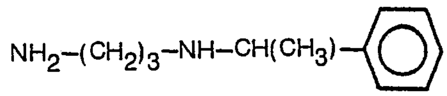

and 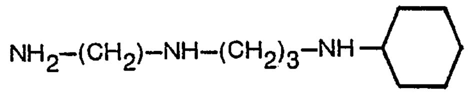

8. A pharmaceutical composition as claimed in either of claims 1 or 3 in which the amplifying compound is caffeine.

9. A pharmaceutical composition according to claim 1, 2 or 3 wherein the acid amide and the amplifying compound are a combined preparation for simultaneous, separate or sequential use in antibiotic and/or antitumor therapy.

**Patentansprüche**

1. Pharmazeutische Zusammensetzung, die ein Tallysomycinsäureamid, bei dem die Amin-Einheit aus Verbindungen ausgewählt ist, die die Strukturformeln aufweisen:

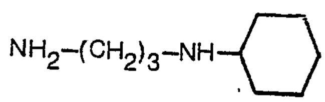

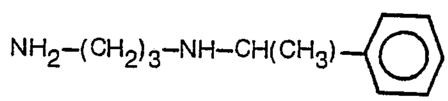

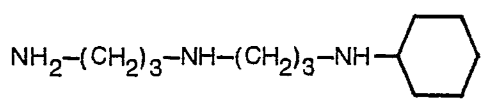

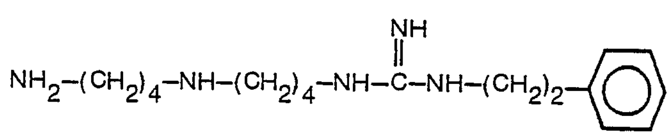

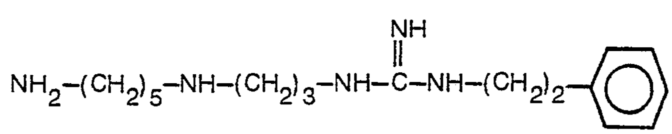

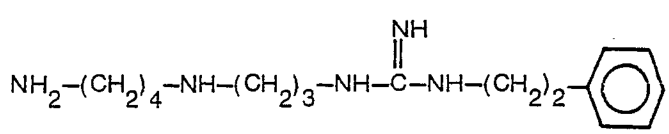

sowie eine verstärkende Verbindung, die aus Coffein, Dimethylthiocyanin und Pyronin Y ausgewählt ist, umfaßt.

2. Pharmazeutische Zusammensetzung, die ein Amin der Phleomycinsäure, der Bleomycinsäure oder der Tallysomycinsäure, bei denen die Amin-Einheit aus den Verbindungen ausgewählt ist, die die Strukturformeln aufweisen:

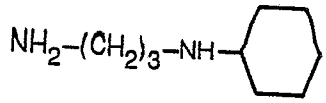

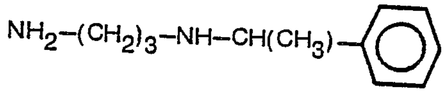

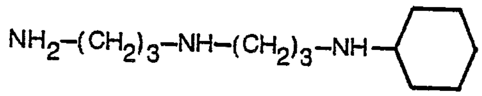

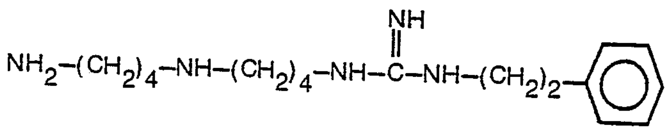

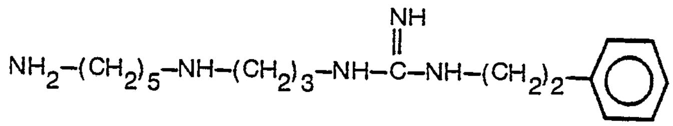

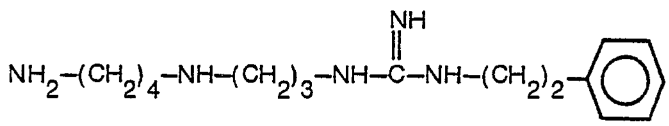

sowie die verstärkende Verbindung Dimethylthiocyanin umfaßt.

3. Pharmazeutische Zusammensetzung zur Verwendung als Antitumor-Mittel, die ein Amid der Phleomycinsäure, der Bleomycinsäure oder der Tallysomycinsäure, bei denen die Amin-Einheit aus Verbindungen ausgewählt ist, die die Strukturformeln aufweisen:

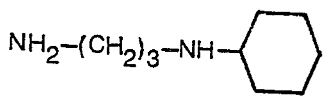

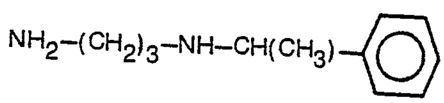

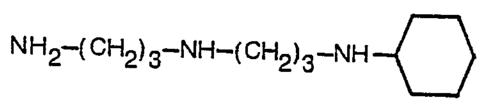

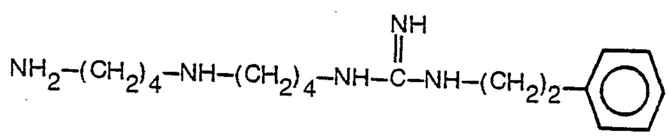

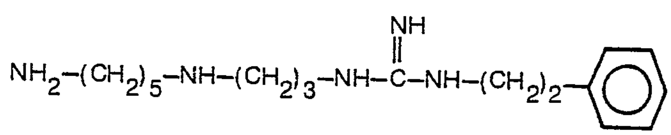

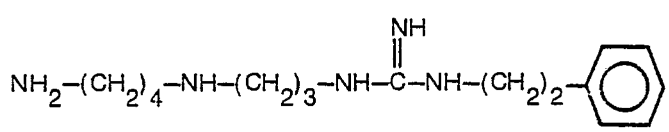

sowie eine verstärkende Verbindung, die aus Coffein und Dimethylthiocyanin ausgewählt ist, umfaßt.

4. Pharmazeutische Zusammensetzung nach Anspruch 2 oder 3, bei der das Amid ein Amid der Phleomycinsäure ist.

5. Pharmazeutische Zusammensetzung nach Anspruch 2 oder 3, bei der das Amid ein Amid der Bleomycinsäure ist.

6. Pharmazeutische Zusammensetzung nach einem beliebigen vorausgehenden Anspruch, bei der das Amid und die verstärkende Verbindung für die Verabreichung in einem Gewichtsverhältnis von 1:0,002 bis 1:30 aufbereitet sind.

7. Pharmazeutische Zusammensetzung nach einem beliebigen vorausgehenden Anspruch, bei der die Amin-Einheit aus Verbindungen ausgewählt ist, die die Strukturformeln aufweisen:

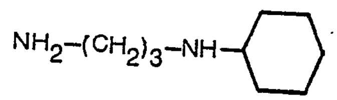

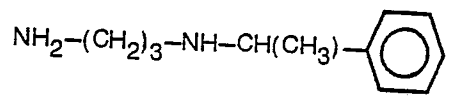 und

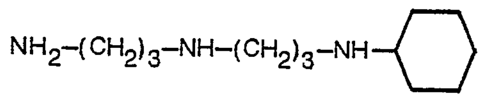

8. Pharmazeutische Zusammensetzung nach Anspruch 1 oder 3, bei der die verstärkende Verbindung Coffein ist.

9. Pharmazeutische Zusammensetzung nach Anspruch 1, 2 oder 3, bei der das Säureamid und die verstärkende Verbindung ein Kombinationspräparat für die gleichzeitige, die getrennte oder die schrittweise Anwendung in der Antibiotika- oder Antitumor-Therapie bilden.

**Revendications**

1. Composition pharmaceutique comprenant un amide de l'acide tallysomycinique dans lequel la partie amide est choisie parmi des composés ayant les formules dévelopées suivantes:

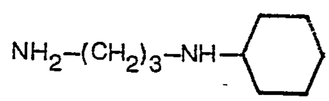

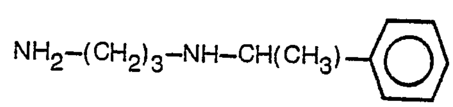

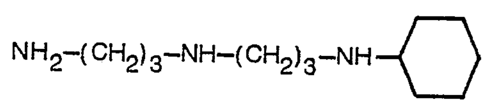

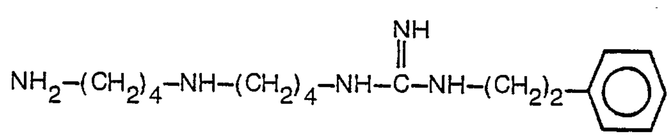

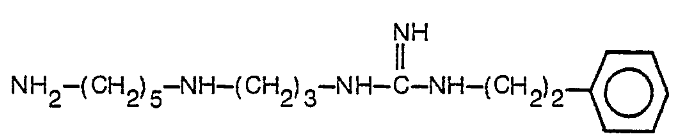

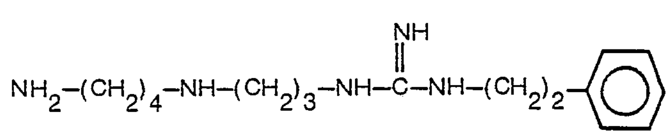

et un composé amplifiant choisi parmi la caféine, la diméthylthiocyanine et la Pyronine Y.

2. Composition pharmaceutique comprenant un amide de l'acide phléomycinique, de l'acide bléomycinique ou de l'acide tallysomycinique, dans lequel la partie amine est choisie parmi les composés ayant les formules dévelopées:

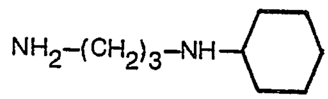

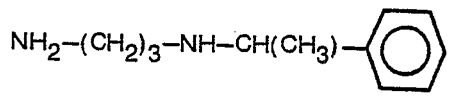

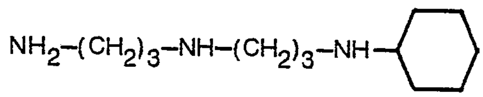

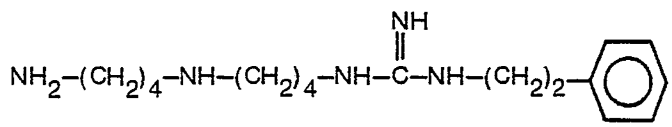

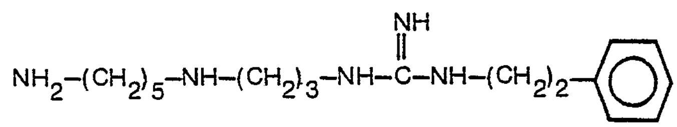

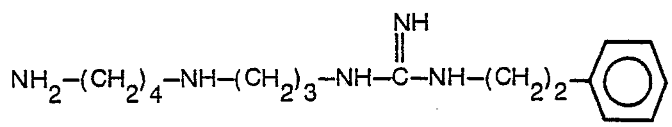

et le composé amplificateur diméthylthiocyanine.

3. Composition pharmaceutique pour l'utilisation comme agent antitumoral comprenant un amide de l'acide phléomycinique, de l'acide bléomycinique ou de l'acide tallysomycinique, dans lequel la partie amine est choisie parmi des composés ayant les formules développées:

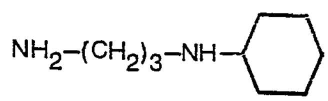

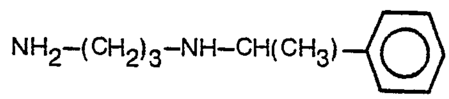

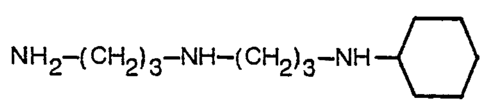

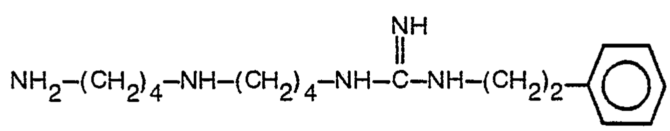

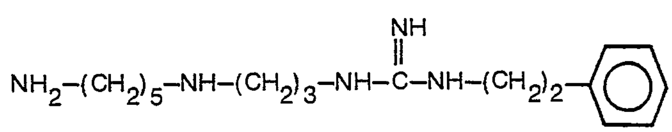

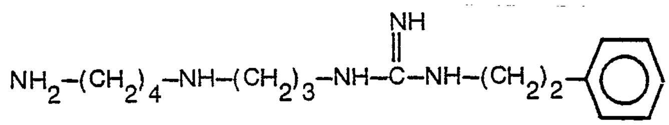

et un composé amplifiant choisi parmi la caféine, la diméthylthiocyanine.

4. Composition pharmaceutique suivant l'une quelconque des revendications 2 ou 3, dans laquelle l'amide est un amide de l'acide phléomycinique.

5. Composition pharmaceutique suivant l'une quelconque des revendications 2 ou 3, dans laquelle l'amide est un amide de l'acide bléomycinique.

6. Composition pharmaceutique suivant l'une quelconque des revendications précédentes, dans laquelle l'amide et le composé amplificateur sont adaptés pour l'administration dans un rapport pondéral de 1:0,002 à 1:3.

7. Composition pharmaceutique suivant l'une quelconque des revendications précédentes, dans laquelle la partie amine est choisi parmi des composés ayant les formules développées:

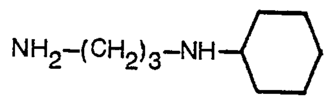

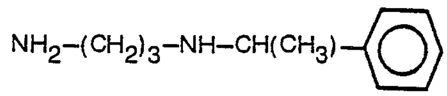

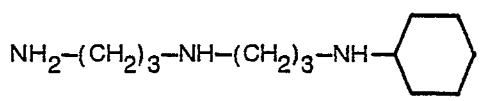

8. Composition pharmaceutiquement suivant l'une quelconque des revendications 1 ou 3, dans laquelle le composé amplificateur est la caféine.

9. Composition pharmaceutique suivant l'une quelconque des revendications 1, 2 ou 3, dans laquelle l'amide et le composé amplificateur sont une préparation combinée pour l'utilisation simultanée, séparée ou successive dans un traitement antibiotique et/ou antitumoral.